(19) Europäisches Patentamt
European Patent Office
Office européen des brevets

(11) EP 1 161 434 B1

(12) FASCICULE DE BREVET EUROPEEN

(45) Date de publication et mention de la délivrance du brevet: **29.01.2003 Bulletin 2003/05**

(21) Numéro de dépôt: **00907746.2**

(22) Date de dépôt: **01.03.2000**

(51) Int Cl.[7]: **C07D 491/22**, A61K 31/44, A61P 25/00 // (C07D491/22, 311:00, 221:00, 221:00), C07D209:00

(86) Numéro de dépôt international: **PCT/FR00/00502**

(87) Numéro de publication internationale: **WO 00/053608 (14.09.2000 Gazette 2000/37)**

(54) **DERIVES DE PYRIDOPYRANOAZEPINES, LEUR PREPARATION ET LEUR APPLICATION EN THERAPEUTIQUE**

PYRIDOPYRANOAZEPINDERIVATE, IHRE HERSTELLUNG UND THERAPEUTISCHE VERWENDUNG

PYRIDOPYRANOAZEPINE DERIVATIVES, PREPARATION AND THERAPEUTIC USE

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **05.03.1999 FR 9902784**

(43) Date de publication de la demande: **12.12.2001 Bulletin 2001/50**

(73) Titulaire: **SANOFI-SYNTHELABO**
**75013 Paris (FR)**

(72) Inventeurs:
- **GALLI, Frédéric**
  **F-92420 Vaucresson (FR)**
- **JEGHAM, Samir**
  **F-34980 Montferrier-sur-Lez (FR)**
- **LOCHEAD, Alistair**
  **F-94220 Charenton (FR)**
- **SAMSON, Axelle**
  **F-75012 Paris (FR)**

(74) Mandataire: **Ludwig, Jacques**
**Sanofi-Synthélabo**
**Service Brevets**
**174, avenue de France**
**75013 Paris (FR)**

(56) Documents cités:
**FR-A- 2 761 072**

- **G. NORDVALL ET AL.: "3-(2-Benzofuranyl)quinuclidin-2-ene derivatives: novel muscarinic antagonists" JOURNAL OF MEDICINAL CHEMISTRY., vol. 39, no. 17, 1996, pages 3269-77, XP002123211 AMERICAN CHEMICAL SOCIETY. WASHINGTON., US ISSN: 0022-2623**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 161 434 B1

Printed by Jouve, 75001 PARIS (FR)

## Description

La présente invention a pour objet des composés de formule générale (I)

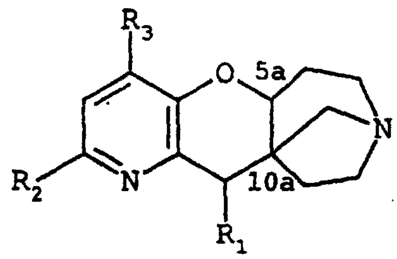

(I)

dans laquelle

$R_1$ représente un atome d'hydrogène, un groupe $(C_1-C_4)$alkyle, un groupe phényl$(C_1-C_4)$alkyle, un groupe phénylhydroxy$(C_1-C_4)$alkyle, un groupe furanyl$(C_1-C_4)$alkyle ou un groupe furanylhydroxy$(C_1-C_4)$alkyle,

$R_2$ représente soit un atome d'hydrogène ou d'halogène ou un groupe trifluorométhyle, cyano, hydroxy, nitro, acétyle, $(C_1-C_6)$alkyle, $(C_1-C_6)$alcoxy ou un groupe de formule générale $NR_4R_5$ dans laquelle $R_4$ représente un atome d'hydrogène ou un groupe $(C_1-C_4)$alkyle ou $(C_1-C_4)$alcanoyle et $R_5$ représente un atome d'hydrogène ou un groupe $(C_1-C_4)$ alkyle, ou bien $R_4$ et

$R_5$ forment, avec l'atome d'azote qui les porte, un cycle en $C_4-C_7$, soit un groupe phényle ou naphtyle éventuellement substitué par un atome d'halogène ou un groupe trifluorométhyle, trifluorométhoxy, cyano, hydroxy, nitro, acétyle, $(C_1-C_6)$alkyle, $(C_1-C_6)$alcoxy, méthylènedioxy lié aux positions 2 et 3 ou 3 et 4 du cycle phényle, ou phényle, et

$R_3$ représente un atome d'hydrogène ou d'halogène ou un groupe $(C_1-C_4)$ alkyle.

Les composés de formule générale (I) peuvent exister à l'état de bases ou de sels d'addition à des acides. Par ailleurs, les atomes en positions 5a et 10a étant asymétriques, un composé peut exister sous forme d'isomères géométriques et optiques purs ou de mélanges de ces derniers.

Conformément à l'invention, on peut préparer les composés de formule générale (I) par un procédé illustré par le schéma qui suit.

Schéma

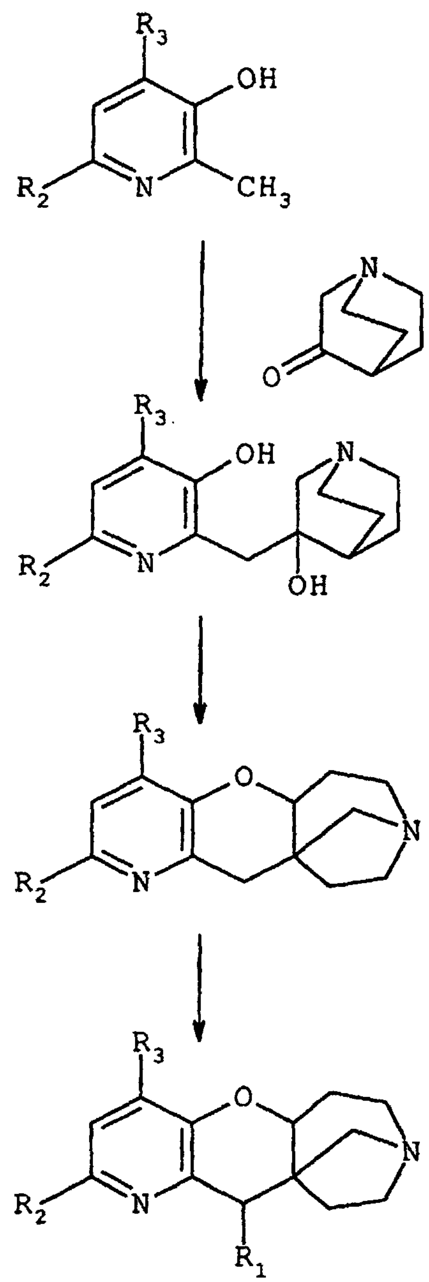

(II)

(III)

(IV)

(Ia)

(I)

On fait réagir un 2-méthylpyridin-3-ol de formule générale (II), dans laquelle $R_2$ et $R_3$ sont tels que définis ci-dessus, avec un alkyllithium, puis on condense l'intermédiaire ainsi obtenu avec la 1-azabicyclo[2.2.2]octan-3-one de formule (III), à basse température et dans un solvant aprotique tel que le tétrahydrofurane.

On obtient un composé de formule générale (IV), sur lequel on peut, si on le désire, introduire ou modifier les substituants $R_2$ et $R_3$ selon toutes méthodes connues de l'homme du métier.

On soumet ensuite le composé de formule générale (IV) à une deshydratation, qui s'accompagne d'un réarrangement, en milieu acide, par exemple l'acide méthanesulfonique ou l'acide sulfurique à haute température.

On obtient un composé de formule générale (Ia), sur lequel on peut modifier les substituants $R_2$ et $R_3$ et/ou introduire le substituant $R_1$ selon toutes méthodes connues de l'homme du métier.

Les composés de départ de formules (II) et (III) sont disponibles dans le commerce ($R_2$=$R_3$=H) ou peuvent être préparés selon des méthodes connues.

Les exemples qui vont suivre illustrent la préparation de quelques composés de l'invention. Les microanalyses

élémentaires, et les spectres I.R. et R.M.N., ainsi que les spectres de diffraction aux rayons X, dans certains cas, confirment les structures des composés obtenus.
Les numéros indiqués entre parenthèses dans les titres des exemples correspondent à ceux de la 1ère colonne du tableau donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

Exemple 1 (Composé N°1).

Chlorhydrate de (*trans*)-5a,6,7,9,10,11-hexahydro-8,10a-méthanopyrido[2',3':5,6]pyrano[2,3-d]azépine (2:1).

1.1. 3-[(3-Hydroxypyridin-2-yl)méthyl]-1-azabicyclo[2.2.2]octan-3-ol.

Dans un ballon tricol de 2000 ml on introduit 52,9 g (484 mmoles) de 2-méthyl-3-hydroxypyridine en solution dans 1300 ml de tétrahydrofurane sous argon. On refroidit la solution à -56°C et on ajoute 750 ml (975 mmoles) d'une solution de 1-méthylpropyllithium 1,3 M dans le cyclohexane, goutte à goutte, en 3 h, en maintenant la température inférieure à -50°C. A la fin de l'addition on laisse la température remonter jusquà -4°C en 45 min puis on refroidit à nouveau à -58°C pour ajouter 60,6 g (484 mmoles) de 1-azabicyclo[2.2.2]octan-3-one en solution dans 250 ml de tétrahydrofurane, goutte à goutte en 40 min. On laisse la température remonter jusqu'à l'ambiante et on maintient l'agitation pendant 20 h. On refroidit le mélange réactionnel à 4°C et on hydrolyse par addition de 110 ml d'une solution aqueuse d'acide chlorhydrique à 36%. On ajoute 400 ml d'eau, on décante les deux phases et on extrait la phase organique par de l'eau. On réunit les phases aqueuses, on refroidit le milieu à 4°C et on ajoute une solution aqueuse concentrée d'hydroxyde de sodium jusqu'à pH 8,4. On filtre le précipité obtenu et on le sèche sous vide à 80°C.
On obtient ainsi 62,5 g de produit.
Point de fusion : 270-272°C.

1.2. Chlorhydrate de (trans)-5a,6,7,9,10,11-hexahydro-8,10a-méthanopyrido[2',3':5,6]pyrano[2,3-*d*]azépine (2:1).

Dans un ballon de 50 ml on introduit 2,34 g (10 mmoles) de 3-[(3-hydroxypyridin-2-yl)méthyl]-1-azabicyclo [2.2.2]octan-3-ol en solution dans 10 ml d'acide méthanesulfonique et on chauffe à 180°C pendant 48h.
On refroidit le milieu réactionnel et on le verse sur de la glace. On alcalinise par addition d'une solution aqueuse concentrée d'hydroxyde de sodium et on extrait au chloroforme. On sèche la phase organique sur sulfate de magnésium et on la concentre sous pression réduite. On purifie le résidu par chromatographie sur colonne de gel de silice en éluant avec un mélange 90/10/1 de chloroforme, méthanol et ammoniaque. On obtient le produit sous forme de base que l'on salifie par addition d'une solution d'acide chlorhydrique dans l'éthanol. On isole 1,55 g de chlorhydrate.
Point de fusion : >300°C.

Exemple 2 (Composé N°2).

Chlorhydrate de (5a*S*,10a*R*)-5a,6,7,9,10,11-hexahydro-8,10a-méthanopyrido[2',3':5,6]pyrano[2,3-*d*]azépine (2:1).

2.1. (3*R*,5*R*)-(-)-O,O'-dibenzoyl-L-tartrate de (5a*S*,10a*R*)-5a,6,7,9,10,11-hexahydro-8,10a-méthanopyrido[2',3':5,6] pyrano[2,3-*d*]azépine (1:2).

Dans un ballon de 500 ml on introduit 15,335 g (0,0709 mole) de (*trans*)-5a,6,7,9,10,11-hexahydro-8,10a-méthanopyrido[2',3':5,6]pyrano[2,3-*d*]azépine dans 50 ml d'acétate d'éthyle. On ajoute une solution de 50,83 g (0,142 mole) d'acide (3*R*,5*R*)-(-)-O,O'-dibenzoyl-L-tartrique dans 50 ml d'acétate d'éthyle, on évapore le solvant sous pression réduite et on dissout le résidu dans 885 ml d'un mélange 7/3 d'eau et d'éthanol au reflux. Après refroidissement on collecte les cristaux obtenus par filtration et pn les recristallise dans 50 ml de propan-2-ol à chaud.
Après refroidissement on obtient 13,7g de cristaux.
Point de fusion : 145-148°C ; $[\alpha]_D^{20}$ = -104,3° (c=0,5, MeOH).

2.2. Chlorhydrate de (5a*S*,10a*R*)-5a,6,7,9,10,11-hexahydro-8,10a-méthanopyrido[2',3':5,6]pyrano[2,3-*d*]azépine (2: 1).

Le traitement du composé précédent avec une solution aqueuse de carbonate de potassium suivi d'une extraction par du dichlorométhane permet d'obtenir 3,1 g (0,0143 mole) de composé sous forme de base.
Point de fusion : 69-71°C.

$[\alpha]_D^{20}$ = -75,4° (C=1, MeOH).
Dans un ballon de 50 ml on met en solution cette base dans 10 ml d'éthanol, on ajoute 6 ml (0,030 mole) d'une solution d'acide chlorhydrique à 6 M dans le propan-2-ol, on concentre le mélange à sec sous pression réduite, on reprend le résidu dans 40 ml de propan-2-ol, on chauffe jusqu'au reflux et on ajoute 5 ml d'éthanol. Après refroidissement on collecte les cristaux obtenus par filtration et on les sèche sous pression réduite.
On obtient 3,4 g de cristaux blancs.
Point de fusion : 330°C ; $[\alpha]_D^{20}$ = -85,3° (c=1, MeOH).

Exemple 3 (Composé N°4).

(*trans*)-2-Bromo-5a,6,7,9,10,11-hexahydro-8,10a-méthanopyrido[2',3':5,6]pyrano[2,3-d]azépine.

3.1. 3-[(6-Bromo-3-hydroxypyridin-2-yl)méthyl]-1-azabicyclo[2.2.2]octan-3-ol.

Dans un ballon de 1000 ml on introduit 52,23 g (0,223 mole) de 3-[(3-hydroxypyridin-2-yl)méthyl]-1-azabicyclo [2.2.2]octan-3-ol en suspension dans 500 ml d'eau à température ambiante. On ajoute 26,7g (0,669 mole) d'hydroxyde de sodium en solution dans 350 ml d'eau et 26,5 g (0,223 mole) de bromure de potassium puis on agite jusqu'à dissolution complète avant d'ajouter 11,5 ml (0,223 mole) de brome, goutte à goutte, en 2 h.
On laisse agiter pendant 18 h à température ambiante, puis on neutralise le milieu réactionnel par addition de 23 ml d'acide acétique. On le refroidit dans un bain de glace et on filtre le précipité obtenu. On concentre les eaux-mères et on triture le précipité obtenu dans le propan-2-ol, on le filtre et on le rince.
On obtient 27,9 g de produit.
Point de fusion : 215-221°C.

3.2. (*trans*)-2-Bromo-5a,6,7,9,10,11-hexahydro-8,10a-méthanopyrido[2',3':5,6]pyrano[2,3-d]azépine.

Dans un ballon de 100 ml on introduit 6,1 g de 3-[(6-bromo-3-hydroxypyridin-2-yl)méthyl]-1-azabicyclo[2.2.2] octan-3-ol et 50 ml d'acide sulfurique concentré. On chauffe le mélange à 130°C pendant 72 h, puis on le refroidit à température ambiante et on le verse sur de la glace. On alcalinise la phase aqueuse à pH 10 par addition d'une solution aqueuse concentrée d'hydroxyde de sodium et on l'extrait par du chloroforme. On sèche les phases organiques sur sulfate de magnésium et on les concentre sous pression réduite. On purifie le résidu par chromatographie sur colonne de gel de silice en éluant avec un mélange 90/10/4 de dichlorométhane, méthanol et ammoniaque. On obtient 1,2 g de produit.
Point de fusion : 157-159°C

Exemple 4 (Composé N°28).

Bromhydrate de *trans*-(-)-2-bromo-5a,6,7,9,10,11-hexahydro-8,10a-méthanopyrido[2',3':5,6]pyrano[2,3-*d*]azépine (1: 1).

4.1. (3*R*,5*R*)-(-)-O,O'-dibenzoyl-L-tartrate de (5a*S*,10a*R*)-2-bromo-5a,6,7,9,10,11-hexahydro-8,10a-méthanopyrido[2', 3':5,6]pyrano[2,3-d]azépine (1:2).

Dans un ballon de 50 ml on introduit 0,3 g (1 mmole) de (*trans*)-2-bromo-5a,6,7,9,10,11-hexahydro-8,10a-méthanopyrido[2',3':5,6]pyrano[2,3-*d*]azépine en solution dans 10 ml d'acétate d'éthyle, on ajoute 0,358 g (1 mmole) d'acide O,O'-(-)-dibenzoyl-L-tartrique en solution dans 3 ml d'acétate d'éthyle, on évapore le solvant sous pression réduite et on recristallise le résidu dans 5 ml de propan-2-ol chaud. Après refroidissement on collecte les cristaux obtenus par filtration et on les sèche sous vide.
On obtient 0,12 g de cristaux.
Point de fusion : 200°C.
$[\alpha]_D^{20}$ = -106° (C=0,5, MeOH).

4.2. Bromhydrate de *trans*-(-)-2-bromo-5a,6,7,9,10,11-hexahydro-8,10a-méthanopyrido[2',3':5,6]pyrano[2,3-*d*]azépine (1:1).

Le passage à la la base s'effectue par traitement du composé précédent avec une solution aqueuse d'hydroxyde de sodium, suivi d'une extraction par du dichlorométhane.
Dans un ballon de 100 ml on met en solution 0,3 g (1 mmole) de base dans 30 ml de propan-2-ol. On ajoute 0,36 ml

(2 mmoles) d'une solution d'acide bromhydrique à 33% dans l'acide acétique. Après refroidissement à 4°C on collecte les cristaux obtenus par filtration et on les sèche sous vide.
On obtient 0,25 g de cristaux blancs.
Point de fusion : 350-352°C ; $[\alpha]_D^{20}$ = -76,3° (c=0,5, MeOH).

Exemple 5 (Composé N°24).

Chlorhydrate de *trans*-(-)-2-chloro-5a,6,7,9,10,11-hexahydro-8,10a-méthanopyrido[2',3':5,6]pyrano[2,3-*d*]azépine (2:1).

On dissout 0,2 g (0,68 mmole) de *trans*-(-)-2-bromo-5a,6,7,9,10,11-hexahydro-8,10a-méthanopyrido[2',3':5,6]pyrano[2,3-d]azépine dans 4 ml d'une solution aqueuse concentrée d'acide chlorhydrique et on chauffe à 180°C dans un tube scellé pendant 48 h.
On évapore la phase aqueuse et on recristallise le résidu dans le propan-2-ol.
On obtient 0,075 g de cristaux.
Point de fusion : 339-344°C ; $[\alpha]_D^{20}$ = -81° (c=0,5, MeOH).

Exemple 6 (Composé N°27).

Bromhydrate de (trans)-2-cyano-5a,6,7,9,10,11-hexahydro-8,10a-méthanopyrido[2',3':5,6]pyrano[2,3-d]azépine (1:1).

Dans un ballon de 50 ml on met en solution 0,45 g (1,52 mmole) de (*trans*)-2-bromo-5a,6,7,9,10,11-hexahydro-8,10a-méthanopyrido[2',3':5,6]pyrano[2,3-*d*]azépine dans 8 ml de pyridine, on ajoute 0,205 g (2,29 mmoles) de cyanure de cuivre et on chauffe au reflux pendant 30 h.
On ajoute 75 ml de dichlorométhane et on lave la phase organique par 45 ml d'une solution aqueuse saturée de chlorure d'ammonium, puis par 75 ml d'eau. Après séchage et concentration de la phase organique sous pression réduite on obtient 0,22 g de produit attendu. On le met en solution dans le propan-2-ol et on le traite par un équivalent d'acide bromhydrique en solution à 33% dans l'acide acétique. On obtient après refroidissement, collecte des cristaux par filtration et séchage sous vide, 0,21 g de produit.
Point de fusion : 329-332°C.

Exemple 7 (Composé N°10).

Bromhydrate de (*trans*)-2-(4-méthylphényl)-5a,6,7,9,10,11-hexahydro-8,10a-méthanopyrido[2',3':5,6]pyrano[2,3-*d*]azépine (2:1).

Dans un réacteur de 10 ml on introduit 0,3 g (1 mmole) de (*trans*)-2-bromo-5a,6,7,9,10,11-hexahydro-8,10a-méthanopyrido[2',3':5,6]pyrano[2,3-*d*]azépine dans 6 ml de toluène, 0,193 g (1,4 mmole) d'acide 4-méthylphénylboronique, 0,072 g (0,06 mmole) de palladium tétrakis(triphénylphosphine, 1 ml (2 mmoles) de carbonate de sodium en solution aqueuse 2 M et 0,05 ml d'éthanol, et on chauffe le milieu réactionnel au reflux pendant 72 h.
Après décantation on dépose la phase organique sur gel de silice et on élue avec un mélange 97/3/0,3 de dichlorométhane, méthanol et ammoniaque.
On obtient 0,31 g de produit que l'on salifie par deux équivalents d'acide bromhydrique en solution dans l'acide acétique.
Point de fusion : 355°C.

Exemple 8 (Composé N°5).

Chlorhydrate de (*trans*)-11-méthyl-5a,6,7,9,10,11-hexahydro-8,10a-méthanopyrido[2',3':5,6]pyrano[2,3-*d*]azépine (2:1).

Dans un ballon tricol de 100 ml on introduit 0,43 g (2 mmoles) de (*trans*)-5a,6,7,9,10,11-hexahydro-8,10a-méthanopyrido[2',3':5,6]pyrano[2,3-*d*]azépine dans 20 ml de tétrahydrofurane anhydre, on refroidit le milieu réactionnel à -78°C pour ajouter 1,2 ml (3 mmoles) de butyllithium 2,5 M dans l'hexane, goutte à goutte, et on poursuit l'agitation à -78°C pendant 30 min.
On ajoute 0,19 ml (3 mmoles) d'iodométhane et on laisse le mélange se réchauffer lentement jusquà température ambiante avant d'ajouter 100 ml d'eau et d'extraire par du dichlorométhane. On sèche la phase organique sur sulfate de magnésium, on l'évapore sous pression réduite et on purifie le résidu par chromatographie sur colonne de gel de silice en éluant avec un mélange 90/10/1 de dichlorométhane, méthanol et ammoniaque. On traite le produit obtenu

avec deux équivalents d'acide chlorhydrique en solution dans le propan-2-ol et isole par filtration 0,15 g de cristaux. Point de fusion : >330°C.

Exemple 9 (Composé N°9).

Bromhydrate de (*trans*)-α-furan-3-yl-5a,6,7,9,10,11-hexahydro-10a*H*-8,10a-méthanopyrido[2',3':5,6]pyrano[2,3-*d*]azépine-11-méthanol (2:1).

On traite 0,43 g (2 mmoles) de (*trans*)-5a,6,7,9,10,11-hexahydro-8,10a-méthanopyrido[2',3':5,6]pyrano[2,3-*d*]azépine avec du furane-3-carboxaldéhyde dans les conditions décrites dans l'exemple 8.
Après salification par 2 équivalents d'acide bromhydrique dans l'acide acétique on obtient 0,3 g de composé.
Point de fusion : 69-73°C avec décomposition.

Exemple 10 (Composé N°26).

Bromhydrate de (*trans*)-2,4-dibromo-5a,6,7,9,10,11-hexahydro-8,10a-méthanopyrido[2',3':5,6]pyrano[2,3-*d*]azépine (1:1).

10.1. 3-[(4,6-dibromo-3-Hydroxypyridin-2-yl)méthyl]-1-azabicyclo[2.2.2]octan-3-ol.

Dans un ballon de 2000 ml on introduit une solution de 24 g (0,426 mole) d'hydroxyde de potassium dans 600 ml d'eau, on ajoute 50,0 g (0,213 mole) de 3-[(3-hydroxypyridin-2-yl)méthyl]-1-azabicyclo[2.2.2]octan-3-ol, puis, au goutte à goutte, en 40 min, une solution de 10,93 ml (0,213 mole) de brome et 152,4 g (1,280 mole) de bromure de potassium dans 600 ml d'eau, et on agite le mélange à température ambiante pendant 16 h.
On en ajuste le pH à 7,5 par addition d'acide acétique, on l'agite pendant 1 h. On le filtre, on sèche le solide obtenu, on le reprend dans 1000 ml d'éthanol, et on chauffe la suspension obtenue pendant 2 h.
Après refroidissement on recueille le précipité par filtration et on le sèche.
On obtient 21,24 g de solide.
Point de fusion : 260-265°C.

10.2. Bromhydrate de (*trans*)-2,4-dibromo-5a,6,7,9,10,11-hexahydro-8,10a-méthanopyrido[2',3':5,6]pyrano[2,3-*d*]azépine (1:1).

Dans un ballon de 500 ml on introduit 10 g (25 mmoles) de 3-[(4,6-dibromo-3-hydroxypyridin-2-yl)méthyl]-1-azabicyclo[2.2.2]octan-3-ol, on ajoute 150 ml d'acide sulfurique concentré et 3,6 g (25 mmoles) de pentoxyde de phosphore et on chauffe le mélange à 150°C pendant 48 h.
On le refroidit, on le verse sur 300 g de glace, on ajuste le pH à 10 par addition d'ammoniaque et on l'extrait avec du chloroforme. On sèche la phase organique sur sulfate de sodium, on la filtre, on évapore le solvant sous pression réduite et on purifie le résidu par chromatographie sur colonne de gel de silice en éluant avec un mélange 98/2/0,2 de chloroforme, méthanol et ammoniaque.
Après salification du solide obtenu par un équivalent d'acide bromhydrique dans l'acide acétique on obtient 3,53 g de bromhydrate.
Point de fusion : 320°C avec décomposition.

Le tableau qui suit illustre les structures chimiques et les propriétés physiques de quelques composés de l'invention. Dans les colonnes "$R_1$" et "$R_2$", "$C_6H_5$", "$C_6H_4$" et "$C_6H_3$" désignent, respectivement, des groupes phényles non substitué, monosubstitués ou disubstitués. Les substituants et leurs positions sont indiqués. "$C_4H_3O$" désigne un groupe furan-3-yle. "2-$C_{10}H_7$" désigne un groupe naphtalén-2-yle.
La colonne "5a,10a" indique la configuration des centres chiraux 5a et 10a et "+/-" désigne un racémate.
Dans la colonne "Sel", "-" désigne un composé à l'état de base, "HCl" désigne un chlorhydrate, "HBr" désigne un bromhydrate, "dbL" désigne un dibenzoyl-L-tartrate et "dbD" désigne un dibenzoyl-D-tartrate. Les rapports molaires acide:base sont indiqués.
Dans la colonne "F (°C)", "(d)" indique un point de fusion avec décomposition.

Tableau

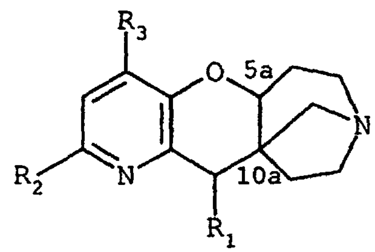

(I)

| N° | $R_1$ | $R_2$ | $R_3$ | 5a,10a | Sel | F (°C) | $[\alpha]_D^{20}$ (°) |
|---|---|---|---|---|---|---|---|
| 1 | H | H | H | (+/-) | HCl 2:1 | >300 | - |
| 2 | H | H | H | *S,R* | dbL 1:1 | 145-148 | -104,3 c=0,5, MeOH |
| | | | | | - | 69-71 | -75,4 c=1, MeOH |
| | | | | | HCl 2:1 | 330 | -85,3 c=1, MeOH |
| 3 | H | H | H | *R,S* | - | 69-71 | +75 c=1, MeOH |
| 4 | H | Br | H | (+/-) | - | 157-159 | - |
| 5 | $CH_3$ | H | H | (+/-) | HCl 2:1 | >330 | - |
| 6 | $CH_2CH_3$ | H | H | (+/-) | HBr 2:1 | 170 (d) | - |
| 7 | $CH_2C_6H_5$ | H | H | (+/-) | HBr 2:1 | 274-276 | - |
| 8 | $CH(OH)C_6H_5$ | H | H | (+/-) | HCl 2:1 | 231-233 | - |
| 9 | $CH(OH)-3-C_4H_3O$ | H | H | (+/-) | HBr 2:1 | 69-73 (d) | - |
| 10 | H | $C_6H_4-4-CH_3$ | H | (+/-) | HBr 2:1 | 355 | - |
| 11 | H | $C_6H_5$ | H | (+/-) | HBr 1:1 | 350-359 | - |

| N° | $R_1$ | $R_2$ | $R_3$ | 5a,10a | Sel | F (°C) | $[\alpha]_D^{20}$ (°) |
|---|---|---|---|---|---|---|---|
| 12 | H | $C_6H_4$-4-$OCF_3$ | H | (+/-) | - | 120-121 | - |
| 13 | H | $C_6H_4$-4-$CF_3$ | H | (+/-) | - | 120 | - |
| 14 | H | $C_6H_4$-3-$NO_2$ | H | (+/-) | - | 203 (d) | - |
| 15 | H | $C_6H_4$-3-$COCH_3$ | H | (+/-) | HBr 2:1 | 260 | - |
| 16 | H | $C_6H_3$-3,4-($OCH_2O$) | H | (+/-) | HBr 2:1 | 310 | - |
| 17 | H | $C_6H_3$-3,5-$(CF_3)_2$ | H | (+/-) | - | 102-103 | - |
| 18 | H | $C_6H_4$-4-F | H | (+/-) | HBr 2:1 | 302 | - |
| 19 | H | $C_6H_4$-4-$C_6H_5$ | H | (+/-) | HBr 2:1 | 350 | - |
| 20 | H | 2-$C_{10}H_7$ | H | (+/-) | - | 182 | - |
| 21 | H | Br | H | - | dbL 1:1 | 200 | -106 c=0,7, MeOH |
| 22 | H | Br | H | + | dbD 1:1 | 214-217 | +108 c=0,4, MeOH |
| 23 | H | Cl | H | (+/-) | HCl 2:1 | 280-281 | - |
| 24 | H | Cl | H | - | HCl 2:1 | 339-344 | -81 c=0,5, MeOH |
| 25 | H | Cl | H | + | - | 82-85 | +94,1 c=0,5, $CHCl_3$ |
| 26 | H | Br | Br | (+/-) | HBr 1:1 | 320 (d) | - |
| 27 | H | CN | H | (+/-) | HBr 1:1 | 329-332 | - |
| 28 | H | Br | H | - | HBr 1:1 | 350-352 | -76,3 c=0,5, MeOH |

Les composés de l'invention ont fait l'objet d'essais qui ont mis en évidence leurs propriétés thérapeutiques.

Ainsi ils ont été étudiés quant à leur affinité vis-à-vis des récepteurs nicotiniques contenant la sous-unité $\alpha_4\beta_2$ selon les méthodes décrites par Anderson et Arneric, *Eur. J. Pharmacol* (1994) **253** 261, et par Hall et coll., *Brain Res.* (1993) **600** 127.

On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 g pendant 10 min. On élimine le culot, et on centrifuge le surnageant à 20000 g pendant 20 min à 4°C. On récupère le culot et on l'homogénéise à l'aide d'un broyeur Polytron™ dans 15 volumes d'eau bidistillée à 4°C, puis on le centrifuge à 8000 g pendant 20 min. On élimine le culot et on centrifuge le surnageant et la couche de peau ("buffy coat") à 40000 g pendant 20 min, on récupère le culot, on le remet en suspension dans 15 ml d'eau bidistillée à 4°C et on le centrifuge encore une fois à 40000 g avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de [$^3$H]cytisine 1 nM dans un volume final de 500 µl de tampon, en présence ou en absence de composé à tester. On arrête la réaction par filtration sur des filtres Whatman GF/B™ préalablement traités avec de la polyéthylèneimine, on rince les filtres avec deux fois 5 ml de tampon à 4°C, et on mesure la radioactivité retenue sur le filtre par scintigraphie liquide.On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [$^3$H]cytisine, puis on calcule la $CI_{50}$, concentration de composé qui inhibe 50% de la liaison spécifique.

Les $CI_{50}$ des composés de l'invention les plus affins se situent entre 0,08 et 1 µM.

Les composés de l'invention ont aussi été étudiés quant à leur affinité vis-à-vis des récepteurs nicotiniques contenant la sous-unité $\alpha$7, selon les méthodes décrites par Marks et Collins, *J.Pharmacol.Exp.Ther*. (1982) **22** 554 et Marks et al., *Mol. Pharmacol.* (1986) **30** 427.

On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4°C, puis on le centrifuge à 1000 g pendant 10 min. On élimine le culot, et on centrifuge le surnageant à 8000 g pendant 20 min à 4°C. On récupère le culot et on l'homogénéise à l'aide d'un broyeur Polytron™ dans 15 volumes d'eau bidistillée à 4°C, puis on le centifuge à 8000 g pendant 20 min. On élimine le culot et on centrifuge le surnageant et la couche de peau ("buffy coat") à 40000 g pendant 20 min. On récupère le culot, on le remet en suspension avec 15 volumes d'eau bidistillée à 4°C et on le centrifuge encore une fois à 40000 g pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis le membranes sont incubées pendant 60 min à 37°C, à l'obscurité, en présence de 50 µl de [$^3$H]$\alpha$-bungarotoxine 1 nM dans un volume final de 250 µl de tampon HEPES 20 mM à 0,05% de polyéthylèneimine. On arrête la réaction par filtration sur des filtres Whatman GF/C™ préalablement traités pendant 3 heures avec de la polyéthylèneimine à 0,5%. On rince les filtres avec deux fois 5 ml de tampon à 4°C, et on mesure la radioactivité retenue sur chaque filtre par scintigraphie liquide. On détermine la liaison non spécifique en présence de $\alpha$-bungarotoxine à 1 µM final ; la liaison non spécifique représente environ 60% de la liaison totale récupérée sur le filtre. Pour chaque concentration de composé étudié on détermine le pourcentage d'inhibition de la liaison spécifique de [$^3$H]$\alpha$-bungarotoxine, puis on calcule la $CI_{50}$, concentration de composé qui inhibe 50% de la liaison spécifique.

Les $CI_{50}$ des composés de l'invention se situent entre 1 et 20 µM.

Les composés de l'invention ont également été étudiés quant à leur affinité vis-à-vis des récepteurs nicotiniques périphérique de type ganglionnaire selon la méthode décrite par Houghtling et al., *Mol. Pharmacol.* (1995) **48** 280-287. La capacité d'un composé à déplacer la [$^3$H]-epibatidine des membranes de glandes surrénales de boeuf mesure son affinité pour ce récepteur.

On décongèle des glandes surrénales de boeuf conservées à -80°C, et on les homogénéise à l'aide d'un broyeur Polytron™ dans 20 volumes de tampon Tris-HCl 50 mM à pH 7,4 à 4°C, puis on les centifuge à 35000 g pendant 10 min. On élimine le surnageant et on remet le culot en suspension dans 30 volumes de tampon Tris-HCl 50 mM à 4°C et on réhomogénéise avant de recentrifuger à 35000 g pendant 10 min. On reprend le dernier culot dans 10 volumes de tampon Tris-HCl à 4°C. On fait incuber 100 µl de membrane soit 10 mg de tissu frais à 24°C pendant 3 h en présence de 50 µl de [$^3$H]-epibatidine 0,66 nM final dans un volume final de 250 µl de tampon, en présence ou en absence de composé à tester. On arrête la réaction par dilution des échantillons avec du tampon Tris-HCl 50 µM pH 7,4 à 4°C puis on filtre sur de filtres Whatman GF/C™ préalablement traités pendant 3 heures avec de la polyéthylèneimine à 0,5%. On rince les filtres 2 fois par 5 ml de tampon et on mesure la radioactivité retenue sur le filtre par scintigraphie liquide. On détermine la liaison non spécifique en présence de (-)nicotine 2 mM final ; la liaison non spécifique représente 30 à 40% de la liaison totale récupérée sur le filtre. Pour chaque concentration de composé étudié on détermine le pourcentage d'inhibition de la liaison spécifique de [$^3$H]-epibatidine, puis on calcule la $CI_{50}$, concentration de composé qui

inhibe 50% de la liaison spécifique.
Les $CI_{50}$ des composés de l'invention les plus affins se situent entre 9 et 20 $\mu$M.

Les résultats des essais qui précèdent montrent que certains composés de l'invention sont des ligands sélectifs pour les sous-unités $\alpha_4\beta_2$ du récepteur nicotinique.

Enfin les composés de l'invention ont fait l'objet d'essais *in vivo* qui ont mis en évidence leurs propriétés thérapeutiques.
Ainsi, par exemple, ils ont été étudiés dans le modèle de la plaque chauffante, selon la méthode de Eddy et Leimbach, *J. Pharmacol. Exp. Ther*. (1953) **107** 385-393 dans le but de rechercher et quantifier un éventuel effet analgésique.
Des souris de 20 à 30 g sont soumis à un stimulus thermique par contact des pattes avec une plaque maintenue à la température constante de 57,5°C par un bain-marie thermostaté. On mesure le temps de réaction à la douleur qui se manifeste par un léchage de pattes ou un saut. Ainsi, après le délai de prétraitement effectué par voie sous-cutanée ou orale (chaque lot étant constitué de huit animaux pour un même prétraitement), les souris sont déposées individuellement sur la plaque et le temps de réaction à la douleur est mesuré. L'animal est retiré de la plaque immédiatement après la manifestation de la douleur. Le temps maximum d'exposition au stimulus est de 30 s.
On exprime pour chaque lot le temps moyen de réaction accompagné de l'erreur standard à la moyenne (e.s.m). Une analyse de variance non paramétrique (Kruskal-Wallis), est effectuée sur l'ensemble du lot. Un test de Wilcoxon permet la comparaison de chaque lot traité au lot témoin. Les différences sont considérées comme statistiquement significatives au seuil 5%.
Ce temps de réaction est significativement augmenté par les analgésiques principalement à effets centraux.
Les composés de l'invention montrent une activité dans ce test aux doses comprises entre 3 et 30 mg/kg par voie intrapéritonéale ou orale.

Ces résultats suggèrent l'utilisation des composés dans le traitement ou la prévention des désordres liés à un dysfonctionnement des récepteurs nicotiniques, notamment au niveau du système nerveux central ou du système gastro-intestinal.

Au niveau du système nerveux central ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multi-infarct dementia, MID).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des accidents vasculaires cérébraux et des épisodes hypoxiques cérébraux. Ils peuvent être utilisés dans les cas de pathologies psychiatriques : schizophrénie, dépression, anxiété, attaques de panique, comportements compulsifs et obsessionnels.
Ils peuvent prévenir les symptômes dus au sevrage au tabac, à l'alcool, aux différentes substances induisant une dépendance, telles que cocaïne, LSD, cannabis, benzodiazépines.
Enfin ils peuvent être utiles pour le traitement de la douleur.

Au niveau du système gastro-intestinal les composés de l'invention pourraient être utiles dans le traitement de la maladie de Crohn, de la colite ulcéreuse, du syndrome du côlon irritable et de l'obésité.

A cet effet les composés de l'invention peuvent être présentés sous toutes formes de compositions appropriées à l'administration entérale, parentérale ou transdermique, telles que comprimés, dragées, gélules, capsules, suspensions ou solutions buvables ou injectables telles que sirops ou ampoules, timbres transdermiques ("patch"), etc, associés à des excipients convenables, et dosés pour permettre une administration journalière de 0,01 à 20 mg/kg.

## Revendications

1. Composé, sous forme d'isomère géométrique ou optique pur ou de mélange de tels isomères, de formule générale (I)

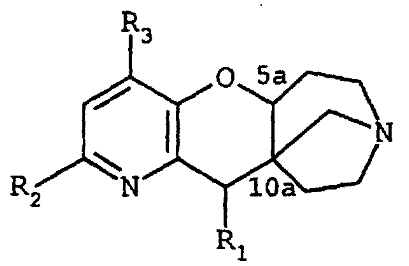

(I)

dans laquelle

$R_1$ représente un atome d'hydrogène, un groupe $(C_1-C_4)$alkyle, un groupe phényl$(C_1-C_4)$alkyle, un groupe phénylhydroxy$(C_1-C_4)$alkyle, un groupe furanyl$(C_1-C_4)$alkyle ou un groupe furanylhydroxy$(C_1-C_4)$alkyle,

$R_2$ représente soit un atome d'hydrogène ou d'halogène ou un groupe trifluorométhyle, cyano, hydroxy, nitro, acétyle, $(C_1-C_6)$alkyle, $(C_1-C_6)$alcoxy ou un groupe de formule générale $NR_4R_5$ dans laquelle $R_4$ représente un atome d'hydrogène ou un groupe $(C_1-C_4)$alkyle ou $(C_1-C_4)$alcanoyle et $R_5$ représente un atome d'hydrogène ou un groupe $(C_1-C_4)$alkyle, ou bien $R_4$ et

$R_5$ forment, avec l'atome d'azote qui les porte, un cycle en $C_4-C_7$, soit un groupe phényle ou naphtyle éventuellement substitué par un atome d'halogène ou un groupe trifluorométhyle, trifluorométhoxy, cyano, hydroxy, nitro, acétyle, $(C_1-C_6)$alkyle, $(C_1-C_6)$alcoxy, méthylènedioxy lié aux positions 2 et 3 ou 3 et 4 du cycle phényle, ou phényle, et

$R_3$ représente un atome d'hydrogène ou d'halogène ou un groupe $(C_1-C_4)$ alkyle,

à l'état de base ou de sel d'addition à un acide.

2. Procédé de préparation de composés selon la revendication 1, **caractérisé en ce qu'**on soumet un composé de formule générale (IV)

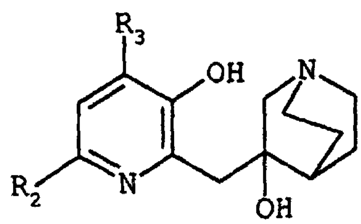

dans laquelle $R_2$ et $R_3$ sont tels que définis dans la revendication 1, à une deshydratation en milieu acide suivie d'un réarrangement à haute température, pour obtenir un composé de formule générale (Ia)

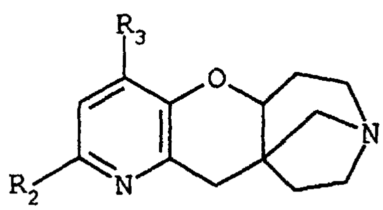

sur lequel, si on le désire, on modifie les substituants $R_2$ et $R_3$ et/ou on introduit un substituant $R_1$ tel que défini dans la revendication 1.

3. Médicament **caractérisé en ce qu'**il consiste en un composé selon la revendication 1.

4. Composition pharmaceutique **caractérisée en ce qu'**elle contient un composé selon la revendication 1, associé à un excipient.

## Patentansprüche

1. Verbindung, in Form des reinen geometrischen oder optischen Isomeren oder einer Mischung solcher Isomeren, der allgemeinen Formel (I)

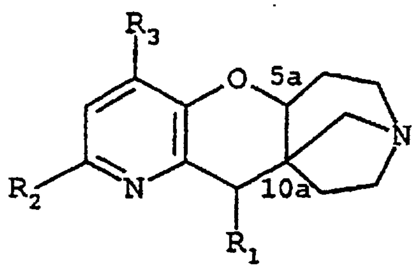

(I)

in der
$R_1$ ein Wasserstoffatom, eine ($C_1$-$C_4$)-Alkylgruppe, eine Phenyl-($C_1$-$C_4$)-alkylgruppe, eine Phenylhydroxy-($C_1$-$C_4$)-alkylgruppe, eine Furanyl-($C_1$-$C_4$)-alkylgruppe oder eine Furanylhydroxy-($C_1$-$C_4$)-alkylgruppe.
$R_2$ entweder ein Wasserstoffatom oder ein Halogenatom oder eine Trifluormethylgruppe, Cyanogruppe, Hydroxygruppe, Nitrogruppe, Acetylgruppe, ($C_1$-$C_6$)-Alkylgruppe, ($C_1$-$C_6$)-Alkoxygruppe oder eine Gruppe der allgemeinen Formel $NR_4R_5$, in der $R_4$ ein Wasserstoffatom oder eine ($C_1$-$C_4$)-Alkylgruppe oder ($C_1$-$C_4$)-Alkanoylgruppe und $R_5$ ein Wasserstoffatom oder eine ($C_1$-$C_4$)-Alkylgruppe darstellen, oder $R_4$ und $R_5$ gemeinsam mit dem sie tragenden Stickstoffatom einen $C_4$-$C_7$-Ring bilden, oder eine Phenylgruppe oder eine Naphthylgruppe, die gegebenenfalls durch ein Halogenatom oder eine Trifluormethylgruppe, Trifluormethoxygruppe, Cyanogruppe, Hydroxygruppe, Nitrogruppe, Acetylgruppe, ($C_1$-$C_6$)-Alkylgruppe, ($C_1$-$C_6$)-Alkoxygruppe, an die Positionen 2 und 3 oder 3 und 4 des Phenylrings gebundene Methylendioxygruppe oder Phenylgruppe substituiert ist, und
$R_3$ ein Wasserstoffatom, ein Halogenatom oder eine ($C_1$-$C_4$)-Alkylgruppe bedeuten,
in Form der Base oder des Additionssalzes mit einer Säure.

2. Verfahren zur Herstellung der Verbindungen nach Anspruch 1, **dadurch gekennzeichnet, daß** man eine Verbindung der allgemeinen Formel (IV)

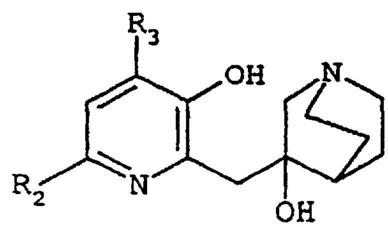

in der $R_2$ und $R_3$ die in Anspruch 1 angegebenen Bedeutungen besitzen, einer Entwässerungsbehandlung in saurem Medium, gefolgt von einer Umlagerung bei hoher Temperatur, unterwirft zur Bildung einer Verbindung der allgemeinen Formel (Ia)

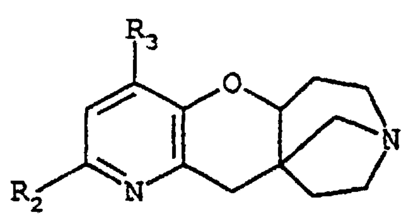

bei der man gewünschtenfalls die Substituenten $R_2$ und $R_3$ modifiziert und/oder einen Substituenten $R_1$, wie er in Anspruch 1 definiert ist, einführt.

**3.** Arzneimittel, **dadurch gekennzeichnet, daß** es aus einer Verbindung nach Anspruch 1 besteht.

**4.** Pharmazeutische Zubereitung, **dadurch gekennzeichnet, daß** sie eine Verbindung nach Anspruch 1 in Kombination mit einem Trägermaterial enthält.

**Claims**

**1.** Compound, in the form of pure geometrical or optical isomers or a mixture of such isomers, of general formula (I)

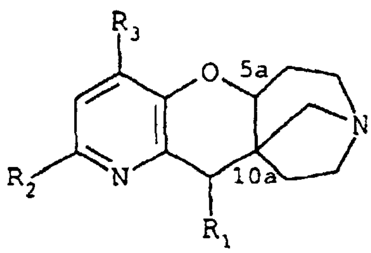

(I)

in which
$R_1$ is a hydrogen atom, a ($C_1$-$C_4$)alkyl group, a phenyl($C_1$-$C_4$)alkyl group, a phenylhydroxy($C_1$-$C_4$)alkyl group, a furanyl($C_1$-$C_4$)alkyl group, or a furanylhydroxy($C_1$-$C_4$)alkyl group,
$R_2$ is either a hydrogen or halogen atom or a trifluoromethyl, cyano, hydroxyl, nitro, acetyl, ($C_1$-$C_6$)alkyl or ($C_1$-$C_6$) alkoxy group or a group of general formula $NR_4R_5$ in which $R_4$ is a hydrogen atom or a ($C_1$-$C_4$)alkyl or ($C_1$-$C_4$) alkanoyl group and
$R_5$ is a hydrogen atom or a ($C_1$-$C_4$)alkyl group, or else $R_4$ and $R_5$ form, with the nitrogen atom which carries them, a $C_4$-$C_7$ ring, or a phenyl or naphthyl group optionally substituted by a halogen atom or a trifluoromethyl, trifluoromethoxy, cyano, hydroxyl, nitro, acetyl, ($C_1$-$C_6$)alkyl, ($C_1$-$C_6$)alkoxy or methylenedioxy group linked in the 2 and 3 or 3 and 4 positions of the phenyl ring, or phenyl, and $R_3$ is a hydrogen or halogen atom or a ($C_1$-$C_4$)alkyl group, in the state of a base or an addition salt to an acid.

**2.** Process for preparation of compounds according to Claim 1, **characterized in that** a compound of general formula (IV)

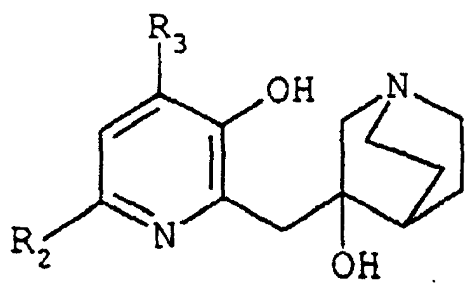

in which $R_2$ and $R_3$ are as defined in Claim 1, is subjected to a dehydration in acid medium followed by a rearrangement at high temperature, to obtain a compound of general formula (Ia)

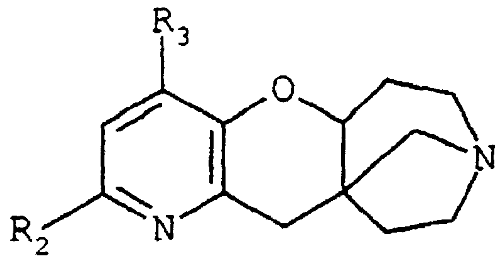

in which, if desired, the substituents $R_2$ and $R_3$ are modified and/or a substituent $R_1$ such as defined in Claim 1 is introduced.

**3.** Medicament **characterized in that** it consists of a compound according to Claim 1.

**4.** Pharmaceutical composition **characterized in that** it contains a compound according to Claim 1, combined with an excipient.